# EUROPÄISCHE PATENTSCHRIFT

(11) **EP 2 157 921 B1**
(45) Veröffentlichungstag und Bekanntmachung des Hinweises auf die Patenterteilung: **02.04.2014**
(21) Anmeldenummer: 08758605.3
(22) Anmeldetag: 17.05.2008
(51) Int. Cl.: A61B 17/16, A61B 17/88, A61B 17/92, A61B 17/22, A61B 17/00, A61H 23/00, A61H 23/02

(54) **MEDIZINISCHES GERÄT ZUR BEHANDLUNG DES MENSCHLICHEN ODER TIERISCHEN KÖRPERS MIT DRUCK- ODER STOßWELLEN**
MEDICAL DEVICE FOR THE TREATMENT OF THE HUMAN OR ANIMAL BODY USING PRESSURE OR IMPACT WAVES
APPAREIL MÉDICAL POUR LE TRAITEMENT DU CORPS HUMAIN OU ANIMAL PAR ONDES DE PRESSION OU ONDES DE CHOC

(30) Priorität: 31.05.2007 DE 202007007921 U
(43) Veröffentlichungstag der Anmeldung: 03.03.2010
(73) Patentinhaber: STORZ MEDICAL AG, 8274 Tägerwilen (CH)
(72) Erfinder: SCHULZ, Manfred, 8274 Tägerwilen (CH); KATONA, Josef, 78315 Radolfszell (DE); MARLINGHAUS, Ernst, H., CH-8598 Bottighofen (CH); NOVAK, Pavel, CH-8234 Stetten (CH); MÜLHAUSER, Peter, Christian, CH-8267 Berlingen (CH)
(74) Vertreter: Witte, Weller & Partner Patentanwälte mbB
(86) Internationale Anmeldenummer: PCT/EP2008/003986
(87) Internationale Veröffentlichungsnummer: WO 2008/145269

(56) Entgegenhaltungen:
- WO-A-97/48353
- DE-A1- 19 524 380
- DE-A1-102005 022 034

## Beschreibung

Die Erfindung betrifft ein medizinisches Gerät zur Behandlung des menschlichen oder tierischen Körpers mit Druck- oder Stoßwellen, mit einer Vorrichtung zur Erzeugung der Druck- oder Stoßwellen, die zumindest ein Applikatorteil und einen pneumatischen Antrieb aufweist, der mittels eines unter Druck stehenden Gases auf eine proximale Eintrittsfläche des Applikatorteils wiederholt Stöße erzeugt, um mit dem Applikatorteil Druck- oder Stoßwellen zu erzeugen, die von dem Applikatorteil über eine distale Austrittsfläche in dem Körper eingekoppelt werden, wobei das Applikatorteil in einem Gehäuse mit einem begrenzten Hub aus einer proximalen Ausgangsstellung nach distal beweglich ist.

Ein solches Gerät ist aus dem Dokument DE 197 25 477 C2 bekannt.

Unter "Behandlung des menschlichen oder tierischen Körpers" ist im Sinne der vorliegenden Erfindung beispielsweise die Behandlung von Weichgewebe, insbeson-Unter "Behandlung des menschlichen oder tierischen Körpers" ist im Sinne der vorliegenden Erfindung beispielsweise die Behandlung von Weichgewebe, insbesondere zur Schmerztherapie, die Behandlung von Knochengewebe, die Zertrümmerung von Körpersteinen, die Entfernung von Plaque in Gefäßen, die Behandlung der Zähne, aber auch das Entfernen von Knochenzement oder das Eintreiben von Knochennägeln oder -drähten zu verstehen.

Das aus dem o.g. Dokument DE 197 25 477 C2 bekannte medizinische Gerät dient zur Behandlung bei Knochenbrüchen, Enthesiopathien, Tendopathien oder auch bei Paradentose. Ein weiteres Einsatzgebiet dieses bekannten Geräts ist die Schmerztherapie im knochennahen Weichteilbereich des Haltungs- und Bewegungsapparates. Alle diese Anwendungen des bekannten medizinischen Geräts sind auch mit dem medizinischen Gerät der vorliegenden Erfindung durchführbar.

Das bekannte Gerät weist als Vorrichtung zur Erzeugung der Druck- oder Stoßwellen außer dem Applikatorteil ein entlang einer Beschleunigungsstrecke pneumatisch beschleunigtes Schlagteil bzw. Projektil auf, das zur Erzeugung der Druck- oder Stoßwellen in dem Applikatorteil wiederholt auf eine proximale Eintrittsfläche des Applikatorteils prallt. Durch den Aufprall des Schlagteils oder Projektils auf den ruhenden Applikator werden in diesem Druck- oder Stoßwellen erzeugt, die über die distale Austrittsfläche des Applikatorteils in den Körper eines Patienten eingekoppelt werden, wenn die distale Austrittsfläche auf die Körperoberfläche des Patienten aufgesetzt wird.

Ein weiteres medizinisches Gerät zur Behandlung des menschlichen oder tierischen Körpers mit Druck- oder Stoßwellen, das dort für die Entfernung von Knochenzement verwendet wird, ist aus dem Dokument WO 97/48353 A1 bekannt. Auch dieses bekannte medizinische Gerät weist als Vorrichtung zur Erzeugung der Druck- oder Stoßwellen ein entlang einer Beschleunigungsstrecke pneumatisch beschleunigtes Schlagteil bzw. Projektil auf, das zur Erzeugung der Druck- oder Stoßwellen wiederholt auf eine proximale Eintrittsfläche eines Applikatorteils prallt, wobei letzteres dort in Form einer Sonde ausgebildet ist, aus deren distaler Spitze die Druck- oder Stoßwellen in den Körper eingekoppelt werden.

Die Ausgestaltung des medizinischen Geräts mit einer Vorrichtung zur Erzeugung der Druck- oder Stoßwellen, die entlang einer Beschleunigungsstrecke ein hin und her bewegtes Schlagteil oder Projektil aufweist, hat den Nachteil, dass das Gerät aufgrund der notwendigen Beschleunigungsstrecke für das Schlagteil bzw. Projektil langbauend ist. Das bekannte Gerät weist entsprechend ein Handstück auf, in dem das Applikatorteil angeordnet ist, das in Form eines länglichen Stabes ausgebildet ist.

Ein weiterer Nachteil des bekannten Geräts besteht darin, dass das Schlagteil oder Projektil und/oder das Applikatorteil einem Verschleiß unterworfen ist, der durch das wiederholte Aufprallen des Projektils auf das Applikatorteil verursacht ist.

Aus dem Dokument DE 195 24 380 A1 ist eine Massageeinrichtung bekannt, die insbesondere in der Entstauungstherapie zur Behandlung von Patienten mit venösen oder lymphatischen Rückflussstörungen ausgebildet ist. Diese Massageeinrichtung ist als Bandage ausgebildet und weist mindestens drei sich über deren Länge erstreckende parallel geführte Druckübertragungsmittel aus elastischem Material auf, die mittels steuerbarer Drucksteuervorrichtungen quer zur Richtung der Bandage zeitlich versetzt beaufschlagbar sind. Durch die intermittierende und quer zur Richtung der Bandage zeitlich versetzte Beaufschlagung der an der Körperoberfläche des Patienten anliegenden Druckübertragungsmittel ergeben sich im Gewebe kurze Druckgradienten, die entlang der zu behandelnden Extremität wandern. Als Druckmittel kann eine Flüssigkeit oder ein gasförmiges Druckmittel eingesetzt werden.

Ferner ist aus dem Dokument US 5,425,735 ein medizinisches Gerät zur Zertrümmerung von Körpersteinen im Harn- und Blasentrakt bzw. zur Zertrümmerung von Plaque in Gefäßen bekannt, bei dem in dem Applikatorteil Druck- oder Stoßwellen mittels eines elektrohydraulischen Wandlers erzeugt werden. Der elektrohydraulische Wandler weist zwei Elektroden auf, die in eine mit Flüssigkeit gefüllte Kammer eintauchen. Durch Beaufschlagen der Elektroden mit Hochspannung werden Funken erzeugt, die in der Flüssigkeit Druck- oder Stoßwellen auslösen, die dann auf die proximale Eintrittsfläche des Applikatorteils wirken. Dieses bekannte Gerät ist wegen des elektrohydraulischen Wandlers konstruktiv aufwändig.

Der Erfindung liegt die Aufgabe zugrunde, ein medizinisches Gerät der eingangs genannten Art dahingehend weiterzubilden, dass die vorstehend genannten Nachteile vermieden werden, dass insbesondere das Gerät kleinbauend und somit handlich ausgeführt werden kann.

Erfindungsgemäß wird diese Aufgabe durch dass in Anspruch 1 definierte medizinische Gerät gelöst, dass das unter Druck stehende Gas in Form von einzelnen Gasdruckstößen unmittelbar auf das Applikatorteil zur Erzeugung der Druck- oder Stoßwellen wirkt.

Bei dem erfindungsgemäßen medizinischen Gerät werden die Druck- oder Stoßwellen mit dem zumindest einen Applikatorteil demnach durch einzelne Gasdruckstöße erzeugt, die unmmittelbar auf die proximale Eintrittsfläche des Applikatorteils wirken. Aufgrund eines solchen Druck- oder Stoßwellenerzeugungsmechanismus entfällt das bei dem bekannten Gerät vorhandene Schlagteil oder Projektil und insbesondere auch die damit verbundene lange Beschleunigungsstrecke für das Schlagteil oder Projektil. Das erfindungsgemäße Gerät kann somit wesentlich kleiner, insbesondere kürzer und damit handlicher ausgeführt werden. Außerdem ist das erfindungsgemäße Gerät gegenüber dem bekannten Gerät wegen des Wegfalls des Schlagteils oder Projektils weniger verschleißanfällig.

Ein weiterer Vorteil des erfindungsgemäßen Geräts besteht darin, dass es aufgrund des Wegfalls einer linearen Beschleunigungsstrecke in einem ergonomischen Design ausgebildet werden kann. Während das bekannte Gerät aufgrund der linearen Beschleunigungsstrecke ein längliches Handstück aufweist, das in weniger ergonomischer Weise in der Art eines Stabs in der Hand gehalten werden muss, kann das erfindungsgemäße Gerät ein Handstück aufweisen, bei dem das Applikatorteil quer zu einer Längsrichtung des Handstücks angeordnet ist. Dies erleichtert die Handhabung des Geräts bei der Applikation der Druck- oder Stoßwellen bei einem Patienten.

Ein weiterer Vorteil des erfindungsgemäßen Geräts besteht darin, dass der Applikator mit einer höheren Pulsfrequenz mit Stößen beaufschlagt werden kann, während die Pulsfrequenz bei dem bekannten Gerät durch die Beschleunigung des Projektilteils limitiert ist.

Das Applikatorteil ist mit einem begrenzten Hub nach distal beweglich in dem Gehäuse gelagert.

Die Auslenkung des Applikatorteils mit begrenztem Hub wirkt insbesondere im Rahmen der Schmerztherapie und der Weichgewebebehandlung zusätzlich zu den Druck- oder Stoßwellen massierend, was die therapeutische Wirkung des Geräts weiter verbessert.

In einer bevorzugten Ausgestaltung ist das Applikatorteil in dem Gehäuse gasdicht gelagert.

Der Vorteil dieser Maßnahme besteht darin, dass die einzelnen Gasdruckstöße im Wesentlichen ohne Druckverlust auf das Applikatorteil wirken, so dass die gesamte Energie der Gasdruckstöße zur Erzeugung der Druck- oder Stoßwellen in dem Applikatorteil genutzt werden kann.

In einer bevorzugten Ausgestaltung ist das Applikatorteil elastisch in die proximale Ausgangsstellung vorgespannt.

Gemäß dieser Ausgestaltung wirken die einzelnen Gasdruckstöße auf das Applikatorteil, das in seiner proximalen Ausgangsstellung ruht, wobei die einzelnen Gasdruckstöße bewirken, dass das Applikatorteil mit dem begrenzten Hub nach distal bewegt wird, wonach das Applikatorteil wieder elastisch in seine proximale Ausgangsstellung zurückbewegt wird. Hierdurch können Schwingungen des Applikatorteils erzeugt werden, die ebenfalls in den Körper des Patienten eingekoppelt werden und therapeutisch wirken. Die Rückstellbewegung des Applikatorteils kann auch aktiv, beispielsweise mit Druckluft, erfolgen.

Vorzugsweise ist der Hub des Applikatorteils kleiner als 10 mm, vorzugsweise kleiner als 5 mm.

In einer weiteren bevorzugten Ausgestaltung beträgt die proximale Eintrittsfläche des Applikatorteils, auf die die Gasdruckstöße wirken, zumindest 0,5 cm², vorzugsweise zumindest 1 cm².

Gemäß dieser Ausgestaltung ist die proximale Eintrittsfläche des Applikatorteils ausreichend großflächig, um mittels der Gasdruckstöße eine große Kraft bei geringer Amplitude der Gasdruckstöße erzielen zu können, wodurch der pneumatische Antrieb mit geringem Druck arbeiten kann, was die Betriebssicherheit des erfindungsgemäßen Geräts erhöht. Die Eintrittsfläche kann dabei auch so groß sein wie die Austrittsfläche oder größer als diese sein, um besonders kraftvolle Stöße mit dem Applikatorteil zu erzielen.

In einer weiteren bevorzugten Ausgestaltung ist das Applikatorteil aus einem Material geringen spezifischen Gewichts, beispielsweise aus einem Leichtmetall, Kunststoff oder Holz gefertigt.

Die Ausführung des zumindest einen Applikatorteils aus einem Material geringen spezifischen Gewichts führt bei einer gegebenen Amplitude der Gasdruckstöße zu einer hohen Geschwindigkeit des Applikatorteils und ermöglicht insbesondere auch eine noch höhere Pulsfrequenz, mit der die Gasdruckstöße auf das Applikatorteil appliziert werden können.

Im Unterschied zu dem bekannten Gerät kann das Applikatorteil insbesondere auch aus Holz gefertigt sein, weil das Applikatorteil wegen des Fehlens eines Projektils oder Schlagteils, das auf das Applikatorteil prallt, auch aus einem weniger schlagfesten Material wie Holz gefertigt werden kann. Ein Applikatorteil aus Holz hat den Vorteil, dass Holz ein hautfreundlicher Naturstoff ist und sich insbesondere beim Aufsetzen auf die Körperoberfläche wegen der geringeren Wärmeleitung und Wärmekapazität von Holz warm anfühlt. Das Äpplikatorteil kann auch aus mehreren Materialien gefertigt sein.

In einer weiteren bevorzugten Ausgestaltung sind die Gasdruckstöße durch zumindest ein Ventil gesteuert.

Hierbei ist von Vorteil, dass über das zumindest eine Ventil die Gasdruckstöße zwangsgesteuert erzeugt werden können. Dadurch kann vorteilhafterweise, wie in einer weiteren bevorzugten Ausgestaltung vorgesehen ist, die Frequenz, die Amplitude und/oder die Dauer der Gasdruckstöße definiert eingestellt und auch verändert werden. Damit kann je nach Applikationsfall die Wirkung der Druck- oder Stoßwellen auf das zu behandelnde Körperareal optimiert werden.

Im Unterschied zu elektromagnetisch angetriebenen Massagegeräten, bei denen die Schwingungen/Vibrationen in der Regel sinusförmig sind, lassen sich über die Steuerung der Gasdruckstöße andere als sinusförmige Schwingungs- oder Vibrationsprofile erzeugen. Außerdem lassen sich im Unterschied zu dem bekannten Gerät, dessen pneumatischer Antrieb ein Schlagteil oder Projektil aufweist, ein wesentlich höherer Gasdurchsatz und dadurch eine höhere Kraft auf das Applikatorteil, auch bei hohen Geschwindigkeiten desselben, erzeugen.

Vorzugsweise ist das zumindest eine Ventil ein Magnetventil.

Ein Magnetventil lässt sich vorteilhafterweise mit sehr kurzen Schaltzeiten öffnen und schließen, wodurch insbesondere hohe Pulsfrequenzen der Gasdruckstöße erzielt werden können, die 30 Hz deutlich übersteigen können.

In einer weiteren bevorzugten Ausgestaltung weist das Gerät ein Handstück auf, das das zumindest eine Applikatorteil aufweist.

Wie bereits oben erwähnt, kann das Handstück im Unterschied zu dem bekannten Gerät in ergonomisch optimierter Formgebung ausgestaltet werden, insbesondere kleinbauend, was die Handhabung des Handstücks bei der Applikation der Druck- oder Stoßwellen bei einem Patienten ergonomischer gestaltet.

Dabei ist es bevorzugt, wenn das Handstück eine Masse von weniger als 700 g aufweist.

Hierbei ist von Vorteil, dass das Handstück eine ermüdungsarme Handhabung erlaubt.

In einer weiteren bevorzugten Ausgestaltung ist das Verhältnis aus Masse des Handstücks und Masse des Applikatorteils größer als 25 : 1, vorzugsweise größer als 50 : 1.

Die Verwendung eines Applikatorteils mit geringer Masse im Vergleich zur Masse des Handstücks hat den Vorteil, dass das Handstück im Betrieb des Geräts nicht oder nur wenig in Schwingungen oder Vibrationen versetzt wird, wodurch die Handhabungsfreundlichkeit des Handstücks weiter verbessert ist.

In einer weiteren bevorzugten Ausgestaltung ist das Handstück mit einem Steuergerät verbindbar, wobei eine Mehrzahl an Handstücken vorgesehen ist, wobei das Steuergerät erkennt, mit welchem Handstück das Steuergerät verbunden ist.

Gemäß dieser Ausgestaltung werden mehrere verschiedene Handstücke bereitgestellt, die jeweils ein Applikatorteil unterschiedlicher Ausgestaltung aufweisen. Wird eines dieser Handstücke mit dem Steuergerät verbunden, erkennt das Steuergerät automatisch mittels einer Erkennungsschaltung, mit welchem Handstück das Steuergerät verbunden ist und kann dann bestimmte Parameterbereiche wie Frequenz, Amplitude und/oder Dauer der Gasdruckstöße voreinstellen. Vorzugsweise lassen sich diese voreingestellten Parameter während des Betriebs des Geräts ändern, um eine optimale Wirkung der Druck- oder Stoßwellen auf das Körperareal zu erzielen.

In einer weiteren bevorzugten Ausgestaltung ist das zumindest eine Ventil im Handstück angeordnet.

Diese Maßnahme hat den Vorteil, dass weniger Gas für den Antrieb benötigt wird, und somit auch kleinere Gasdruckquellen verwendet werden können.

Diese Maßnahme ist auch mit der zuvor genannten Maßnahme von Vorteil, nach der mehrere unterschiedliche Handstücke vorgesehen sind. So können diese unterschiedlichen Handstücke auch mit unterschiedlichen Ventilen ausgestaltet sein, um das jeweilige Handstück entsprechend seines Applikationszweckes optimal an die Frequenz, Amplitude und/oder Dauer der Gasdruckstöße anpassen zu können.

Wie bereits oben erwähnt, ist/sind die Frequenz, die Amplitude und/oder die Dauer der Gasdruckstöße einstellbar.

Dabei ist es weiter bevorzugt, wenn die Frequenz, die Amplitude und/oder der Druck am Handstück einstellbar ist/sind.

Diese Maßnahme erhöht die Bedienungsfreundlichkeit des erfindungsgemäßen Geräts, da die gewünschten Parametereinstellungen durch die Bedienungsperson unmittelbar am Handstück vorgenommen werden können, während eine Einstellung an dem zuvor genannten Steuergerät unter Umständen eine Unterbrechung des Behandlungsvorgangs erfordert, weil das Steuergerät üblicherweise von dem Patienten weiter entfernt angeordnet ist.

In einer weiteren bevorzugten Ausgestaltung ist das Applikatorteil austauschbar.

Diese Maßnahme ist insbesondere dann von Vorteil, wenn nur ein Handstück vorgesehen ist, wobei durch die Austauschbarkeit des Applikatorteils jeweils ein für die betreffende Behandlung optimales Applikatorteil an dem Handstück montiert werden kann.

Dabei ist es bevorzugt, wenn mehrere Applikatorteile vorgesehen sind, die sich hinsichtlich Größe, Masse, Material, Form der distalen Austrittsfläche und/oder Größe der distalen Austrittsfläche unterscheiden.

Mit einer solchen Auswahl an Applikatorteilen kann das für die jeweilige Behandlung optimale Applikatorteil ausgewählt werden.

In einer weiteren bevorzugten Ausgestaltung weist die Austrittsfläche des Applikatorteils eine Form auf, die in unterschiedlichen Richtungen quer zur Längsrichtung unterschiedliche Abmessungen aufweist.

Hierbei ist von Vorteil, dass je nach Orientierung des Applikatorteils in Bezug auf die Körperoberfläche die mit der Körperoberfläche in Berührung kommende Applikatorteilfläche unterschiedlich groß ist, wodurch ebenfalls unterschiedliche Wirkungen der Druck- oder Stoßwellen im zu behandelnden Körperareal erzielt werden können.

Dabei kann die Austrittsfläche beispielsweise oval oder ellipsenförmig sein.

Die Austrittsfläche des Applikatorteils kann auch rund sein und eine konvexe Wölbung aufweisen.

In einer weiteren bevorzugten Ausgestaltung ist das Gerät schallgedämpft.

Der Vorteil dieser Maßnahme besteht darin, dass das erfindungsgemäße Gerät im Betrieb leise ist und die Behandlung nicht durch laute Geräusche beeinträchtigt ist.

In einer weiteren bevorzugten Ausgestaltung ist am Handstück ein Auslöseelement zum Auslösen der einzelnen Gasdruckstöße für Einzelbetrieb und/oder Dauerbetrieb angeordnet.

Durch diese Maßnahme wird die Bedienungsfreundlichkeit des erfindungsgemäßen Geräts weiter verbessert. Der Gasdruck kann dann permanent anstehen und erst bei Betätigung des Auslösers werden die einzelnen Gasdruckstöße erzeugt.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, sofern diese Merkmale oder Merkmals kombinationen ünter die in den Ansprüchen definierte Erfindung fallen.

Ein Ausführungsbeispiel der Erfindung ist in der Zeichnung dargestellt und wird mit Bezug auf diese hiernach näher beschrieben. Es zeigen:
- Figur 1: ein medizinisches Gerät zur Behandlung des menschlichen oder tierischen Körpers mittels Druck- oder Stoßwellen in einer Seitenansicht;
- Figur 2: das Gerät in Figur 1 in einer Vorderansicht; und
- Figur 3: einen Längsschnitt durch das Gerät in Figur 1, wobei Teile des Geräts durch ein Blockschaltbild dargestellt sind.

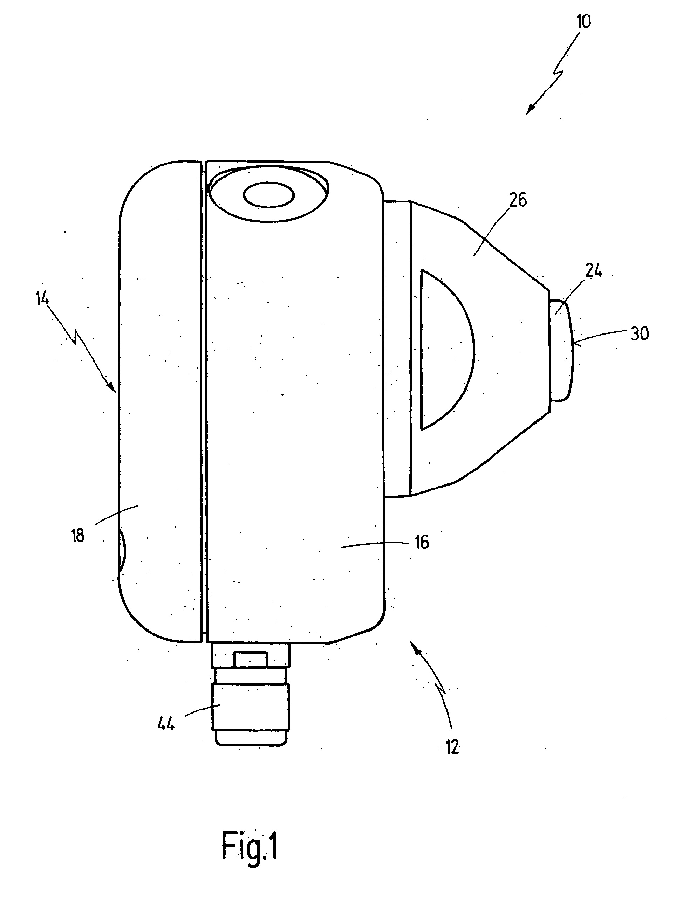

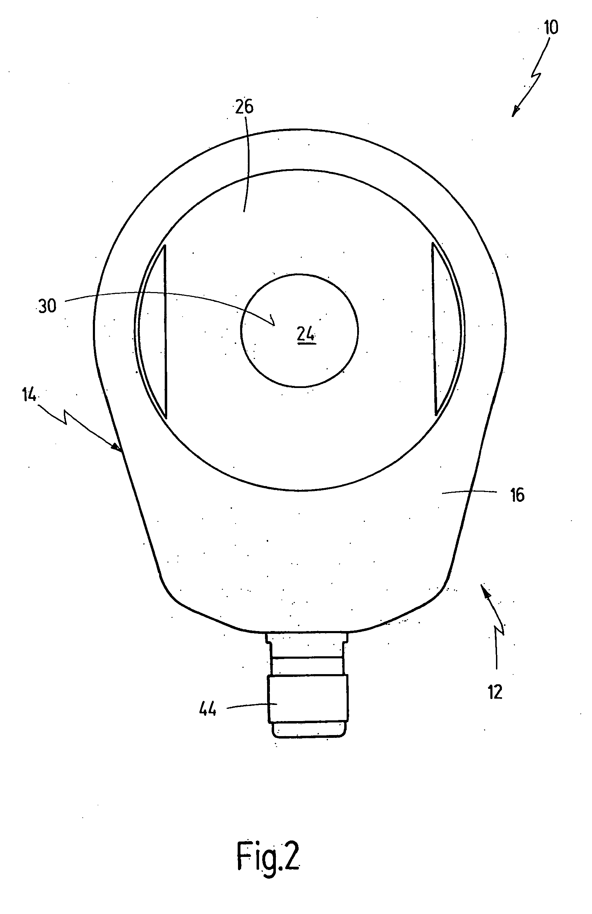

In Figuren 1 bis 3 ist ein mit dem allgemeinen Bezugszeichen 10 versehenes medizinisches Gerät zur Behandlung des menschlichen oder tierischen Körpers mit Druck- oder Stoßwellen dargestellt. Das Gerät 10 dient insbesondere für die Schmerztherapie oder allgemein zur Behandlung biologischen Gewebes im Körper eines Patienten.

Das Gerät 10 weist ein Handstück 12 auf, das eine kompakte Bauweise aufweist, insbesondere im Wesentlichen L-förmig ausgestaltet ist.

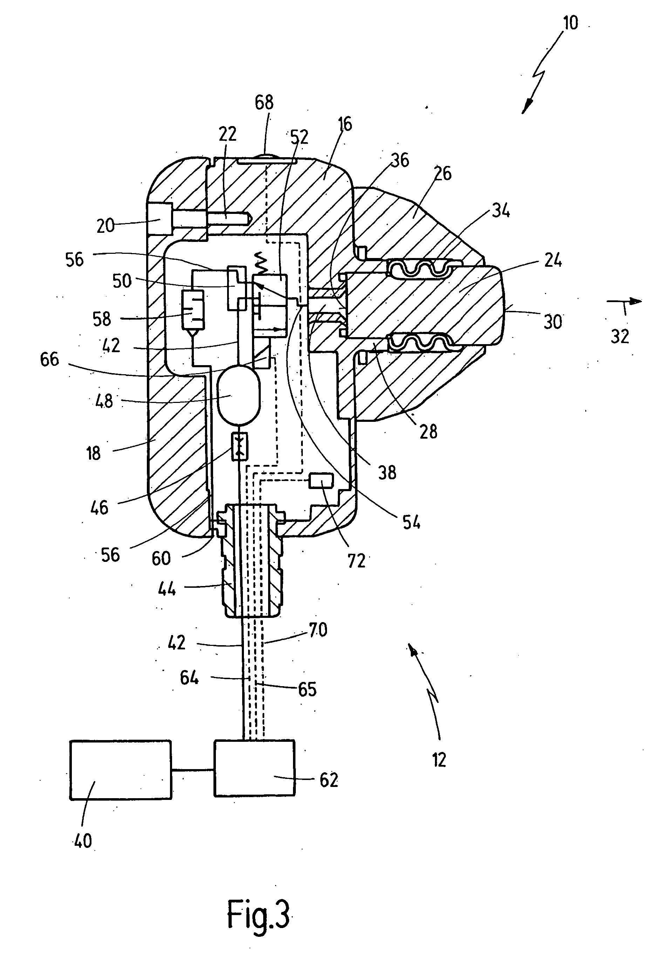

Das Handstück 12 weist ein Gehäuse 14 auf, das in dem gezeigten Ausführungsbeispiel durch ein Gehäusehauptteil 16 und einen Gehäusedeckel 18 gebildet wird, wobei der Gehäusedeckel 18 lösbar mit dem Gehäusehauptteil 16 verbunden ist. Gemäß Figur 3 ist in dem Gehäusedeckel 18 eine Schraubenaufnahme 20 und in dem Gehäusehauptteil 16 eine Gewindebohrung 22 vorgesehen, so dass der Gehäusedeckel 18 mit dem Gehäusehauptteil 16 verschraubt werden kann.

Das Gerät 10 weist weiterhin eine Vorrichtung zur Erzeugung von Druck- oder Stoßwellen auf, die nachfolgend näher beschrieben wird.

Die Vorrichtung zur Erzeugung der Druck- oder Stoßwellen weist zunächst ein Applikatorteil 24 auf, das in dem Gehäuse 14, genauer gesagt in dem Gehäusehauptteil 16, gelagert ist. Zur Fixierung des Applikatorteils 24 an dem Gehäuse 14 ist ein Applikatorhaltekopf 26 vorgesehen, der mit einem Gewindefortsatz 28 des Gehäusehauptteils 16 verschraubt ist. Der Applikatorhaltekopf 26 ist außenseitig konisch ausgebildet.

Von dem Applikatorteil 24 ragt eine distale Austrittsfläche 30 heraus, über die die Druck- oder Stoßwellen in den Körper eines Patienten eingekoppelt werden können.

Das Applikatorteil 24 ist in dem Handstück 12 bzw. dem Gehäuse 14 einschließlich des Applikatorhaltekopfs 26 mit einem begrenzten Hub in Richtung eines Pfeils 32 nach distal beweglich, wobei das Applikatorteil 24 in Figur 3 in seiner proximalen Ausgangsstellung dargestellt ist. Das Applikatorteil 24 ist elastisch über ein Rückstellelement 34 in die proximale Ausgangsstellung vorgespannt. Das Rückstellelement 34 ist im gezeigten Ausführungsbeispiel als balgartige Feder ausgebildet.

Der Hub des Applikatorteils 24 ist kleiner als 10 mm, vorzugsweise kleiner als 5 mm.

Die Vorrichtung zur Erzeugung der Druck- oder Stoßwellen weist weiterhin einen pneumatischen Antrieb auf, der nachfolgend näher beschrieben wird.

Der pneumatische Antrieb verwendet dabei ein unter Druck stehendes Gas, das Stöße in Form von einzelnen Gasdruckstößen auf das Applikatorteil 24 zur Erzeugung der Druck- oder Stoßwellen in dem Applikatorteil 24 ausübt. Die einzelnen Gasdruckstöße wirken dabei unmittelbar auf das Applikatorteil 24, und zwar auf eine proximale Eintrittsfläche 36 des Applikatorteils 24. In unmittelbarer Nähe der proximalen Eintrittsfläche 36 mündet ein Gaszufuhrkanal 38, über den die einzelnen Gasdruckstöße auf die proximale Eintrittsfläche 36 des Applikatorteils 24 gerichtet werden.

Die proximale Eintrittsfläche 36 des Applikatorteils 24 ist großflächig ausgebildet, weist beispielsweise eine Fläche von zumindest 0,5 cm², vorzugsweise von zumindest 1 cm² auf. Während der Gaszufuhrkanal 38 gemäß Figur 3 demgegenüber im Abstand von der Eintrittsfläche 36 einen Querschnitt aufweist, der kleiner ist als die Eintrittsfläche 36, weitet sich der Gaszufuhrkanal 38 in unmittelbarer Nähe der Eintrittsfläche 36 auf, so dass die Gasdruckstöße auf möglichst die gesamte Eintrittsfläche 36 des Applikatorteils 24 wirken.

Damit das über den Gaszufuhrkanal 38 in Form von Gasdruckstößen zugeführte Gas nicht unerwünscht entweicht und somit die gesamte Kraft der Gasdruckstöße auf das Applikatorteil 24 wirken kann, ist das Applikatorteil 24 in dem Gehäuse 14 gasdicht gelagert. Dazu sind nicht näher dargestellte Dichtungen am Applikatorteil 24 bzw. am Gehäuse 14 vorgesehen.

Das Gas wird in einer Gasdruckquelle 40 bereitgestellt. Die Gasdruckquelle 40 kann ein Kompressor zum Komprimieren von Gas, beispielsweise Luft, oder eine mit einem Gas gefüllte Gasflasche oder ein vergleichbares Druckreservoir sein.

Das unter Druck stehende Gas wird über eine Zufuhrleitung 42, beispielsweise einen Druckluftschlauch, dem Handstück 12 zugeführt. Die Zufuhrleitung 42 ist in Figur 3 nur schematisch dargestellt. Zum Anschließen der Zufuhrleitung 42 in Form eines Druckluftschlauches ist an dem Gehäuse 14 des Handstücks 12 ein Anschluss 44 vorgesehen. Im Bereich des Anschlusses 44 befindet sich eine Kupplung 46, die hier schematisch die Trennstelle bzw. die Verbindungsstelle zwischen der Zufuhrleitung 42, die von extern kommt, und den innerhalb des Handstücks 12 befindlichen noch zu beschreibenden weiteren Gasleitungen darstellt.

Im weiteren Verlauf der Gaszufuhr kann ein Reservoir 48 vorgesehen sein, in dem eine Menge an unter Druck stehendem Gas zwischengespeichert werden kann. Von dem Reservoir 48 führt die Zufuhrleitung 42 weiter zu einem Verteiler 50 für Druckluftzufuhr und für Abluft, wobei der Verteiler 50 mit einem Ventil 52, das insbesondere als Magnetventil ausgebildet ist, verbunden ist.

Das Ventil 52 verbindet je nach seinem Schaltzustand eine Gasleitung 54 wechselweise mit der Zufuhrleitung 42 oder einer Gasabfuhrleitung 56.

Die Gasleitung 54 mündet in den Gaszufuhrkanal 38.

Die Gasabfuhrleitung 56 weist einen Schalldämpfer 58 auf, der die bei der Gasströmung entstehenden Geräusche dämpft. Der Schalldämpfer 58 weist eine oder mehrere Kammern auf, die mit einem schalldämpfenden Material gefüllt oder verkleidet sind.

Im weiteren Verlauf mündet die Gasabfuhrleitung 56 aus dem Gehäuse 14 heraus, beispielsweise an einer Stelle 60.

Um das Applikatorteil 24 mit einzelnen Gasdruckstößen zu beaufschlagen, wird das unter Druck stehende Gas über die Zufuhrleitung 42 und den Verteiler 50 bei in Zuführrichtung wiederholt kurzzeitig geöffnetem Ventil 52 stoßartig in den Zufuhrkanal 38 zugeführt, wobei die einzelnen Gasdruckstöße jeweils auf die proximale Eintrittsfläche 36 des Applikatorteils 24 wirken und in dem Applikatorteil 24 Druck- oder Stoßwellen erzeugen, die dann aus der Austrittsfläche 30 austreten und in den Körper eingekoppelt werden. Dazu wird die Austrittsfläche 30 auf die Körperoberfläche aufgesetzt. Die Gasdruckstöße bewirken des Weiteren eine Hubbewegung bzw. Schwingung des Applikatorteils 24.

Die Frequenz, die Amplitude und/oder die Dauer der einzelnen Gasdruckstöße werden durch das Ventil 52 zwangsgesteuert. Nach jedem Gasdruckstoß schließt das Ventil 52 in Zufuhrrichtung und öffnet in Abfuhrrichtung, so dass das Gas nach jedem Gasdruckstoß über die Abfuhrleitung 56 und den Schalldämpfer 58 aus dem Gehäuse 14 entweichen kann.

Das Gerät weist weiterhin ein Steuergerät 62 auf, mit dem das Handstück 12 verbindbar ist. Das Steuergerät 62 steuert alle wesentlichen Funktionen des Handstücks 12. Das Steuergerät 62 steuert auch die Gaszufuhr aus der Gasdruckquelle 40 in die Zufuhrleitung 42.

Über eine erste Steuerleitung bzw. Signalleitung 64 ist das Steuergerät 62 mit einem Steuerkopf 66 des Ventils 52 verbunden, um das Ventil 52 entsprechend zu schalten.

Eine zweite Steuer- bzw. Signalleitung 65 verbindet das Steuergerät 62 mit einem Auslöser 68. Der Auslöser 68 ist am Handstück 12 an einer ergonomisch günstigen Stelle angeordnet, so dass er beispielsweise mit dem Daumen oder Zeigefinger dersel-ben Hand, die das Handstück 12 hält, betätigt werden kann. Der Auslöser 68 ist als Taster ausgebildet.

Durch Betätigen des Auslösers 68 werden die Gasdruckstöße ausgelöst, die auf das Applikatorteil 24 wirken. Durch Betätigen des Auslösers 68 schaltet das Steuergerät 62 das Ventil 52 entsprechend der eingestellten Parameter Frequenz, Amplitude und/oder Dauer der zu erzeugenden Gasdruckstöße. Es kann vorgesehen sein, dass bei Betätigen des Auslösers 68 nur ein einzelner Gasdruckstoß erzeugt wird, vorzugsweise wird jedoch bei dauernd betätigtem Auslöser 68 eine kontinuierliche Folge von Gasdruckstößen erzeugt.

Über eine weitere Steuer- bzw. Signalleitung 70 ist das Steuergerät 62 mit einer Erkennungsschaltung 72 des Handstücks 12 verbunden, wobei die Erkennungsschaltung 72 es ermöglicht, dass das Steuergerät 62 erkennt, mit welchem Handstück das Steuergerät 62 gerade verbunden ist. Im Rahmen der Erfindung ist es nämlich vorgesehen, eine Mehrzahl verschiedener Handstücke 12 bereitzuhalten, die sich beispielsweise durch das jeweilige Applikatorteil 24 und/oder das Ventil 52 unterscheiden. Durch diese Handstückerkennung kann das Steuergerät 62 bestimmte Parameterbereiche voreinstellen, beispielsweise die Dauer, Frequenz und/oder Amplitude der zu erzeugenden Gasdruckstöße, die zur Erzeugung der Druck- oder Stoßwellen in dem jeweiligen Applikator 24 benötigt werden, um eine bestimmte Wirkung derselben zu erzielen.

In nicht dargestellter Weise können jedoch auch am Handstück 12 oder am Steuergerät 62 Bedienelemente vorgesehen sein, mit denen die vorstehend genannten Parameter der Gasdruckstöße auch während des Betriebs des Geräts 10 verändert bzw. eingestellt werden können.

Das Applikatorteil 24 ist an dem Handstück 12 austauschbar angeordnet, wobei zum Austauschen des Applikatorteils 24 lediglich der Applikatorhaltekopf 26 gelöst bzw. abgenommen werden muss.

Das Applikatorteil 24 ist insbesondere aus einem Material geringen spezifischen Gewichts, beispielsweise aus einem Leichtmetall, Kunststoff oder auch aus Holz gefertigt. Als Leichtmetalle kann beispielsweise Aluminium oder Titan, als Kunststoff PMMA (Polymethylmetalycrat), PEEK (Polyetheretherketon) oder auch ein weicherer Kunststoff verwendet werden.

Die Masse des gesamten Handstücks 12 beträgt vorzugsweise weniger als 700 g. Das Verhältnis aus der Masse des Applikatorteils 24 und der Masse des Handstücks ist vorzugsweise kleiner als 1 : 25, vorzugsweise kleiner als 1 : 50.

Im Falle, dass das Applikatorteil 24 austauschbar ist, werden vorzugsweise eine Mehrzahl von Applikatorteilen 24 vorgesehen, die sich hinsichtlich Größe, Masse, Material, Form der distalen Austrittsfläche 30 und/oder Größe der distalen Austrittsfläche 30 unterscheiden. Auf diese Weise kann das für die vorzunehmende Behandlung des Patienten günstigste Applikatorteil 24 verwendet werden.

Außerdem kann vorgesehen sein, dass die Austrittsfläche 30 des Applikatorteils 24 eine Form aufweist, die in unterschiedlichen Richtungen quer zur Längsrichtung (entspricht dem Pfeil 32 in Figur 3) unterschiedliche Abmessungen aufweist, so dass je nach Orientierung des Handstücks 12 bzw. Applikators 24 zur Körperoberfläche des Patienten die Größe der Berührungsfläche der Austrittsfläche 30 mit der Körperoberfläche verschieden ist. Beispielsweise kann die Austrittsfläche 30 oval oder ellipsenförmig ausgebildet sein. Es versteht sich jedoch, dass die Austrittsfläche 30 auch rund ausgebildet sein kann. In dem gezeigten Ausführungsbeispiel ist die Austrittsfläche 30 des Weiteren konvex gewölbt.

Während das in der Zeichnung dargestellte Ausführungsbeispiel des medizinischen Geräts 10 insbesondere für die Schmerztherapie im knochennahen Weichteilbereich geeignet ist, kann das Applikatorteil 24 für andere Verwendungszwecke des Gerätes 10, beispielsweise zur Zertrümmerung von Körpersteinen im Körper, als dünne lange Sonde ausgebildet sein, so dass das Applikatorteil 24 beispielsweise über den Harnweg in den Körper eingeführt werden kann.

Durch eine entsprechende Anpassung des Applikatorteils 24 können weitere Einsatzgebiete in Betracht gezogen werden, beispielsweise die Verwendung zum Eintreiben von Nägeln im Knochen, Abtragen von Knochenzement, usw.

Während das Applikatorteil 24 in dem gezeigten Ausführungsbeispiel einteilig dargestellt ist, versteht es sich, dass das Applikatorteil 24 auch mehrteilig ausgebildet sein kann, beispielsweise auch aus mehreren unterschiedlichen Materialien aufgebaut sein kann.

## Patentansprüche

1. Medizinisches Gerät zur Behandlung des menschlichen oder tierischen Körpers mit Druck- oder Stoßwellen, mit einer Vorrichtung zur Erzeugung der Druck- oder Stoßwellen, die zumindest ein Applikatorteil (24) , ein Gehäuse (14) und einen pneumatischen Antrieb aufweist, der mittels eines unter Druck stehenden Gases auf eine proximale Eintrittsfläche (36) des Applikatorteils (24) wiederholt Stöße erzeugt, um mit dem Applikatorteil (24) Druck- oder Stoßwellen zu erzeugen, die von dem Applikatorteil (24) über eine distale Austrittsfläche (30) in den Körper eingekoppelt werden, wobei das Applikatorteil (24) in dem Gehäuse (14) mit einem begrenzten Hub aus einer proximalen Ausgangsstellung nach distal beweglich gelagert ist, **dadurch gekennzeichnet, dass** das unter Druck stehende Gas in Form von einzelnen Gasdruckstößen unmittelbar auf das Applikatorteil (24) zur Erzeugung der Druck- oder Stoßwellen wirkt.

2. Gerät nach Anspruch 1, **dadurch gekennzeichnet, dass** das Applikatorteil (24) in dem Gehäuse (14) gasdicht gelagert ist.

3. Gerät nach Anspruch 1 oder 2, **dadurch gekennzeichnet, dass** das Applikatorteil (24) elastisch in die proximale Ausgangsstellung vorgespannt ist.

4. Gerät nach einem der Ansprüche 1 bis 3, **dadurch gekennzeichnet, dass** das Applikatorteil (24) aus einem Material geringen spezifischen Gewichts, beispielsweise aus einem Leichtmetall, Kunststoff, Holz, gefertigt ist.

5. Gerät nach einem der Ansprüche 1 bis 4, **dadurch gekennzeichnet, dass** die Gasdruckstöße durch zumindest ein Ventil (52) gesteuert sind.

6. Gerät nach Anspruch 5, **dadurch gekennzeichnet, dass** das zumindest eine Ventil (52) ein Magnetventil ist.

7. Gerät nach einem der Ansprüche 1 bis 6, **dadurch gekennzeichnet, dass** das Gerät (10) ein Handstück (12) aufweist, das das zumindest eine Applikatorteil (24) aufweist.

8. Gerät nach Anspruch 7, **dadurch gekennzeichnet, dass** das Verhältnis aus Masse des Handstücks (12) und Masse des Applikatorteils (24) größer als 25 : 1, vorzugsweise größer als 50 : 1 beträgt.

9. Gerät nach Anspruch 7 oder 8, **dadurch gekennzeichnet, dass** am Handstück (12) ein Auslöser (68) zum Auslösen der Gasdruckstöße im Einzelbetrieb oder Dauerbetrieb angeordnet ist.

10. Gerät nach einem der Ansprüche 7 bis 9, **dadurch gekennzeichnet, dass** das Handstück (12) mit einem Steuergerät (62) verbindbar ist, wobei eine Mehrzahl an Handstücken (12) vorgesehen ist, wobei das Steuergerät (62) erkennt, mit welchem Handstück das Steuergerät (62) verbunden ist.

11. Gerät nach Anspruch 5 oder 6 und nach einem der Ansprüche 7 bis 10, **dadurch gekennzeichnet, dass** das zumindest eine Ventil (52) im Handstück (12) angeordnet ist.

12. Gerät nach einem der Ansprüche 1 bis 11, **dadurch gekennzeichnet, dass** die Frequenz, die Amplitude und/oder die Dauer der Gasdruckstöße einstellbar ist/sind.

13. Gerät nach einem der Ansprüche 7 bis 10 und nach Anspruch 12, **dadurch gekennzeichnet, dass** die Frequenz, die Amplitude und/oder der Druck am Handstück (12) einstellbar ist/sind.

14. Gerät nach einem der Ansprüche 1 bis 13, **dadurch gekennzeichnet, dass** die Austrittsfläche (30) des Applikatorteils (24) eine Form aufweist, die in unterschiedlichen Richtungen quer zur Längsrichtung unterschiedliche Abmessungen aufweist.

15. Gerät nach einem der Anspräche 1 bis 14, **dadurch gekennzeichnet, dass** die Frequenz der Gasdruckstöße größer als 20 Hz, vorzugsweise größer als 30 Hz ist.

## Claims

1. A medical apparatus for treatment of the human or animal body by pressure waves or shock waves, comprising a device designed to generate the pressure waves or shock waves and having at least one applicator part (24), a housing (14) and a pneumatic drive which, by means of a pressurized gas, repeatedly generates impacts on a proximal input face (36) of the applicator part (24) in order, with the applicator part (24), to generate pressure waves or shock waves that are applied from the applicator part (24) into the body by way of a distal output face (30), wherein the applicator part (24) is mounted in the housing in movable fashion from a proximal starting position in distal direction an with a limited stroke, **characterized in that** the pressurized gas acts, in the form of individual gas pressure pulses, directly on the applicator part (24) to generate the pressure waves or shock waves.

2. The apparatus of Claim 1, **characterized in that** the applicator part (24) is mounted in a gas-tight manner in the housing (14).

3. The apparatus of Claim 1 or 2, **characterized in that** the applicator part (24) is pretensioned elastically in a proximal starting position.

4. The apparatus of any one of Claims 1 through 3, **characterized in that** the applicator part (24) is made from a material of low specific weight, for example a light metal, plastic or wood.

5. The apparatus of any one of Claims 1 through 4, **characterized in that** the gas pressure pulses are controlled by at least one valve (52).

6. The apparatus of Claim 5, **characterized in that** the at least one valve (52) is a solenoid valve.

7. The apparatus of any one of Claims 1 through 6, **characterized in that** the apparatus (10) has a handpiece (12) that comprises the at least one applicator part (24).

8. The apparatus of Claim 7, **characterized in that** the ratio of mass of the hand-piece (12) to mass of the applicator part (24) is greater than 25:1, preferably greater than 50:1.

9. The apparatus of Claim 7 or 8, **characterized in that** a trigger (68) for triggering the gas pressure pulses in single operation or continuous operation is arranged on the handpiece (12).

10. The apparatus of any one of Claims 7 through 9, **characterized in that** the handpiece (12) can be connected to a control device (62), in which case a plurality of handpieces (12) are provided, and the control device (62) detects the handpiece to which the control device (62) is connected.

11. The apparatus of Claim 5 or 6 and of any one of Claims 7 through 10, **characterized in that** the at least one valve (52) is arranged in the handpiece (12).

12. The apparatus of any one of Claims 1 through 11, **characterized in that** the frequency, the amplitude and/or the duration of the gas pressure pulses is/are adjustable.

13. The apparatus of any one of Claims 7 through 10 and of Claim 12, **characterized in that** the frequency, the amplitude and/or the pressure can be adjusted on the handpiece (12).

14. The apparatus of any one of Claims 1 through 13, **characterized in that** the output face (30) of the applicator part (24) has a shape that has different dimensions in different directions transverse to the longitudinal direction.

15. The apparatus of any one of Claims 1 through 14, **characterized in that** the frequency of the gas pressure pulses is greater than 20 Hz, preferably greater than 30 Hz.

## Revendications

1. Appareil médical pour le traitement du corps humain ou animal par ondes de pression ou ondes de choc, comportant un dispositif pour générer les ondes de pression ou ondes de choc, qui présente au moins une partie d'applicateur (24), un boîtier (14) et un entraînement pneumatique qui génère de façon répétée des chocs au moyen d'un gaz sous pression sur une surface d'entrée proximale (36) de la partie d'applicateur (24) pour générer avec la partie d'applicateur (24) des ondes de pression ou de choc qui sont couplées par la partie d'applicateur (24) sur une surface de sortie distale (30) dans le corps, dans lequel la partie d'applicateur (24) est placée de façon mobile dans le boîtier (14) avec une course limitée d'une position de sortie proximale à distale, **caractérisé en ce que** le gaz sous pression agit sous la forme d'impulsions de pression de gaz immédiatement sur la partie d'applicateur (24) pour générer les ondes de pression ou de choc.

2. Appareil selon la revendication 1, **caractérisé en ce que** la partie d'applicateur (24) est placée dans le boîtier (14) de façon étanche aux gaz.

3. Appareil selon la revendication 1 ou 2, **caractérisé en ce que** la partie d'applicateur (24) est précontrainte élastiquement dans la position de sortie proximale.

4. Appareil selon l'une des revendications 1 à 3, **caractérisé en ce que** la partie d'applicateur (24) est en un matériau de poids spécifique faible, par exemple en un métal léger, une matière plastique, du bois.

5. Appareil selon l'une des revendications 1 à 4, **caractérisé en ce que** les impulsions de pression de gaz sont commandées par au moins une soupape (52).

6. Appareil selon la revendication 5, **caractérisé en ce qu'**au moins une soupape (52) est une soupape électromagnétique.

7. Appareil selon l'une des revendications 1 à 6, **caractérisé en ce que** appareil (10) présente une pièce à main (12) qui présente au moins une partie d'applicateur (24).

8. Appareil selon la revendication 7, **caractérisé en ce que** le rapport de la masse de la pièce à main (12) à la masse de la partie d'applicateur (24) est supérieur à 25:1, de préférence, supérieur à 50:1.

9. Appareil selon la revendication 7 ou 8, **caractérisé en ce que** sur la pièce à main (12) est disposé un déclencheur (68) pour déclencher les impulsions de pression de gaz en fonctionnement discontinu ou en fonctionnement continu.

10. Appareil selon l'une des revendications 7 à 9, **caractérisé en ce que** la pièce à main (12) peut être reliée à un appareil de commande (62), une pluralité de pièces à main (12) étant prévue, dans lequel l'appareil de commande (62) reconnaît à quelle pièce à main l'appareil de commande (62) est relié.

11. Appareil selon la revendication 5 ou 6, et selon l'une des revendications 7 à 10, **caractérisé en ce qu'**au moins une soupape (52) est disposée dans la pièce à main (12).

12. Appareil selon l'une des revendications 1 à 11, **caractérisé en ce que** la fréquence, l'amplitude et/ou la durée des impulsions de pression de gaz est sont réglable(s).

13. Appareil selon l'une des revendications 7 à 10 et selon la revendication 12, **caractérisé en ce que** la fréquence, l'amplitude et/ou la pression de la pièce à main (12) est/sont réglable(s).

14. Appareil selon l'une des revendications 1 à 13, **caractérisé en ce que** la surface de sortie (30) de la partie d'applicateur (24) présente une forme qui présente des directions différentes perpendiculaires à la direction longitudinale de différentes dimensions.

15. Appareil selon l'une des revendications 1 à 14, **caractérisé en ce que** la fréquence des impulsions de pression de gaz est supérieure à 20 Hz, de préférence supérieure à 30 Hz.
